# EUROPÄISCHE PATENTSCHRIFT

(11) **EP 1 834 659 B1**
(45) Veröffentlichungstag und Bekanntmachung des Hinweises auf die Patenterteilung: **25.02.2009**
(21) Anmeldenummer: 07005467.1
(22) Anmeldetag: 16.03.2007
(51) Int. Cl.: A61M 11/00, A61M 11/02, A61M 11/06, F04B 43/02, F04B 45/04

(54) **Inhalationstherapiegeräte-kompressor**
Inhalation therapy device compressor
Compresseur d'appareils d'inhalation thérapeutique

(30) Priorität: 16.03.2006 DE 102006012174
(43) Veröffentlichungstag der Anmeldung: 19.09.2007
(73) Patentinhaber: PARI GmbH Spezialisten für effektive Inhalation, 82319 Starnberg (DE)
(72) Erfinder: Boehm, Andreas, 86934 Reichling (DE); Luber, Martin, 81545 München (DE)
(74) Vertreter: HOFFMANN EITLE

(56) Entgegenhaltungen:
- CH-A- 281 847
- DE-A1- 19 519 763
- DE-A1- 19 536 860
- DE-A1- 19 947 444
- FR-A1- 2 639 236
- GB-A- 1 224 316

## Beschreibung

Die Erfindung betrifft einen Kompressor für Inhalationstherapiegeräte, bei denen mit Hilfe eines unter Druck zugeführten Gases, in der Regel mit Hilfe von Druckluft, ein therapeutisch wirksames Fluid vernebelt wird.

Wie beispielsweise in EP 0 170 715 A beschrieben, verfügen Inhalationstherapiegeräte dieser Art für die Vernebelung des Fluids über eine druckmittelbetriebene Verneblerdüse, der das Druckmittel und das Fluid zugeführt wird. Dabei beschreibt EP 0 170 715 A eine Zerstäubervorrichtung, bei der der Patient das erzeugte Aerosol über ein Mundstück einatmet. Daneben ist bekannt, für eine Behandlung der Nasenhöhle das erzeugte Aerosol direkt der Nase eines Patienten zuzuführen, wobei DE 102 39 321 B darüber hinausgehend beschreibt, der durch das Druckmittel erzeugten und durch die Nase verlaufenden Aerosolgrundströmung zusätzliche Druckschwankungen zu überlagern, um ein Eindringen des Aerosols in die Nasennebenhöhlen zu erreichen bzw. zu unterstützen. Dafür sind zwei Kompressoren erforderlich, nämlich ein Kompressor für die Erzeugung der Druckluft und ein Kompressor für die Erzeugung der Druckschwankungen. Beide Kompressoren wirken derart zusammen, dass mit Hilfe des eine Verneblerdüse umfassenden Inhalationstherapiegeräts eine Aerosolgrundströmung mit überlagerten Druckschwankungen erzeugt wird. Als Kompressoren werden bei Inhalationstherapiegeräten mit Verneblerdüse gängigerweise elektromotorisch angetriebene Kolben- oder Membrankompressoren eingesetzt, wie sie zum Beispiel in DE 199 27 528 oder in DE 102 39 321 B beschrieben sind. Aus der Patentschrift CH 281 847 A ist eine Luftpumpe für Vernebler von Arzneien bekannt.

Der Aufwand, der mit der Bereitstellung und mit der Handhabung von zwei Kompressoren einhergeht, ist erheblich, so dass bereits vorgeschlagen wurde, nur einen Elektromotor für den Antrieb beider Kompressoren einzusetzen und beide Kompressoren samt antreibendem Elektromotor in einem Gehäuse unterzubringen, wodurch sowohl der Herstellungsaufwand verringert als auch die Handhabung vereinfacht wird.

Vor diesem Hintergrund verfolgt die Erfindung das Ziel, den Aufwand in dem oben geschilderten Bereich weiter zu reduzieren und einen Kompressor bereitzustellen, der einerseits kompakt aufgebaut und wirtschaftlich herstellbar ist und mit dem andererseits sowohl eine Druckgasströmung für die Vernebelung eines therapeutisch wirksamen Fluids als auch Druckschwankungen erzeugbar sind, die einer mit der Druckgasströmung erzeugten Aerosolströmung überlagert werden können. Daneben muss der Kompressor für die therapeutische Anwendung auslegbar sein.

Dieses Ziel wird erreicht mit einem Inhalationstherapiegerätekompressor, der die Merkmale aufweist, wie sie in Patentanspruch 1 definiert sind.

Aufgrund der erfindungsgemäßen Gestaltung weist der Kompressor zwei voneinander getrennte Kompressionsräume auf, von denen der eine der Erzeugung des kontinuierlichen Druckmittelstromes für die Erzeugung einer Aerosolhauptströmung dient, während der andere für die Erzeugung der Druckschwankungen eingesetzt wird. Die gemeinsame Kompressionseinrichtung, beispielsweise ein Kolben, wird von einem Antrieb pendelnd bewegt, sodass durch den ersten Kompressionsraum ein Gas gefördert wird und über den zweiten Kompressionsraum einem Gasvolumen Druckschwankungen aufgeprägt werden.

Der Antrieb ist erfindungsgemäß in einem Kompressionsraum angeordnet, wodurch die Erfindung auf vorteilhafte Weise das zur Verfügung stehende Raumvolumen nutzt, das von dem Kompressor notwendigerweise in Anspruch genommen wird. In einer bevorzugten Ausführungsform wird erfindungsgemäß der Antrieb in dem Kompressionsraum untergebracht, der für die Erzeugung der Druckschwankungen eingesetzt wird. Diese Gestaltung ist vorteilhaft, weil der Kompressionsraum, der für die Erzeugung des kontinuierlichen Druckmittelstroms dient, aufgrund der vergleichsweise hohen Drücke und Fördermengen besonders dicht ausgelegt werden muss. Im Gegensatz dazu ist es einfacher, die für den Antrieb erforderlichen Durchführungen durch die Gehäusewand des Kompressors in den Kompressionsraum hinein, in dem der Antrieb angeordnet ist, dort vorzusehen, wo geringere Drücke auftreten, nämlich in dem Kompressionsraum, in dem die Druckschwankungen erzeugt werden. Grundsätzlich ist aber auch die Anordnung des Antriebs in dem Kompressionsraum möglich, durch den der Druckmittelstrom gefördert wird, wenn eine entsprechende Dichtigkeit an Durchbrüchen und Durchführungen, beispielsweise der Durchführung der Welle des Elektromotors, gewährleistet werden.

Weitere vorteilhafte Ausgestaltungen ergeben sich aus den Unteransprüchen.

Im Folgenden wird die Erfindung anhand von Ausführungsbeispielen und unter Bezugnahme auf die Zeichnungen genauer erläutert. In den Zeichnungen zeigt
- Figur 1: eine schematische Ansicht eines ersten Ausführungsbeispiels;
- Figur 2: eine schematische Ansicht eines zweiten Ausführungsbeispiels;
- Figur 3: eine schematische Ansicht eines dritten Ausführungsbeispiels;
- Figur 4: eine schematische Ansicht eines vierten Ausführungsbeispiels;
- Figur 5: eine Detailansicht einer Gaseinlasseinrichtung mit Einlassventil gemäß der Erfindung;
- Figur 6: eine Detailansicht einer Gasauslasseinrichtung mit Auslassventil gemäß der Erfindung, und
- Figur 7: eine Gaseintrittseinrichtung mit Gaseintrittsventil gemäß der Erfindung.

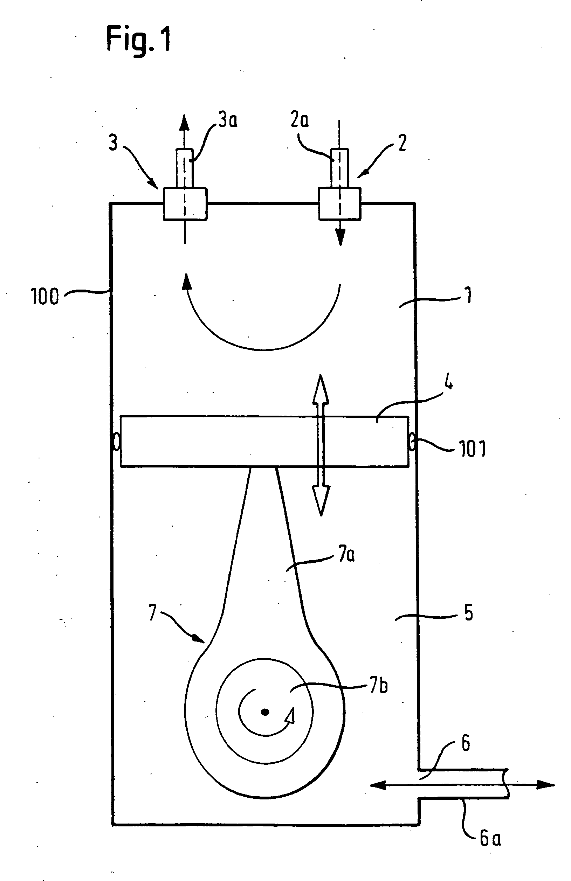

Figur 1 zeigt ein erstes Ausführungsbeispiel des erfindungsgemäßen Inhalationstherapiegerätekompressors. Bei diesem Ausführungsbeispiel umfasst der Kompressor einen ersten Kompressionsraum 1, der von einem Gehäuse 100 des Kompressors umschlossen wird. In dem Gehäuse 100 ist eine Kompressionseinrichtung 4 in Form eines Kolbens vorgesehen, der an seinem äußeren Umfang zum Gehäuse 100 hin mit einer Dichtung 101, beispielsweise aus Teflon, ausgestattet ist und den ersten Kompressionsraum 1 in dem Gehäuse 100 abschließt. In Bezug auf den ersten Kompressionsraum 1 umfasst der in Figur 1 gezeigte Kompressor gemäß der Erfindung ferner eine Gaseinlasseinrichtung 2, die es gestattet, dass ein Gas, beispielsweise Umgebungsluft, in den ersten Kompressionsraum einströmt und die ein Ausströmen des Gases aus dem ersten Kompressionsraum verhindert. Dazu besitzt die Gaseinlasseinrichtung 2 ein Einlassventil, das weiter unten noch genauer beschrieben wird. Für das Ausströmen des Gases aus dem ersten Kompressionsraum 1 ist eine Gasauslasseinrichtung 3 in Bezug auf den ersten Kompressionsraum 1 vorgesehen, die es dem Gas gestattet, aus dem ersten Kompressionsraum auszuströmen und die ein Einströmen des ersten Gases in den ersten Kompressionsraum 1 verhindert. Dazu weist die Gaseinlasseinrichtung 3 ein Einlassventil auf, das weiter unten noch genauer beschrieben wird. Wird der Kolben 4 in dem Gehäuse 100 des erfindungsgemäßen Kompressors hin- und herbewegt, wie durch den Doppelpfeil in Figur 1 angedeutet ist, wird zunächst das erste Gas, beispielsweise Umgebungsluft, durch die Gaseinlasseinrichtung 2 in den ersten Kompressionsraum 1 hinein gefördert und später aufgrund der gegenläufigen Bewegung des Kolbens 4 durch die Gasauslasseinrichtung 3 aus dem Kompressionsraum 1 herausgefördert.

Um den Kolben 4 in dem Gehäuse 100 zu bewegen, ist ein Antrieb vorgesehen, vom dem in Figur 1 eine Bewegungseinrichtung 7 in Form eines Pleuels 7a und einer Exzenter-Scheibe 7b gezeigt ist. Die Exzenter-Scheibe 7b wird von einem Elektromotor (nicht dargestellt) angetrieben, so dass die Rotationsbewegung der Exzenter-Scheibe 7b dazu führt, dass das Pleuel den Kolben 4 in einer Pendelbewegung hin- und herbewegt. Die Pleuelstange kann an dem Kolben 4 fest angebracht sein, wenn die durch die Wechselwirkung des Pleuels 7a mit der Exzenter-Scheibe 7b hervorgerufene Verkippung des Kolbens 4 in dem Gehäuse 100 durch die Dichtung 101 ausgeglichen wird. Andernfalls kann die Pleuelstange auch beweglich an dem Kolben angebracht sein, wobei dann die Dichtung 101 vorzugsweise eine sichere Führung des Kolbens 4 in dem Gehäuse 100 gewährleistet.

Erfindungsgemäß ist die Bewegungseinrichtung 7 bei dem in Figur 1 gezeigten Ausführungsbeispiel in einem zweiten Kompressionsraum 5 angeordnet, der ebenfalls von dem Gehäuse 100 gebildet und von dem Kolben 4 abgeschlossen wird. Mit anderen Worten, die Bewegungseinrichtung 4, hier der Kolben, trennt das Gehäuse 100 des erfindungsgemäßen Kompressors in eine erste Kompressionskammer und eine zweite Kompressionskammer, wobei die Kompressionseinrichtung 4 auf das Gasvolumen beider Kompressionskammern einwirkt, wenn sie von der Bewegungseinrichtung 7 bewegt wird.

In Bezug auf den zweiten Kompressionsraum 5 ist erfindungsgemäß eine Gasdurchtrittsöffnung 6 vorgesehen, aus der ein zweites Gas, bei dem es sich auch um Luft handeln kann, durch die Gasdurchtrittsöffnung 6 aus dem zweiten Kompressionsraum 5 herausströmen kann, wenn der Kolben 4 den zweiten Kompressionsraum verkleinert, und in den zweiten Kompressionsraum 5 hineinströmen kann, wenn der Kolben 4 den zweiten Kompressionsraum vergrößert. Dem in dem zweiten Kompressionsraum 5 befindlichen Gasvolumen und einem gegebenenfalls an den zweiten Kompressionsraum 5 angeschlossenen Gasvolumen, was unten noch genauer erläutert wird, werden Druckschwankungen aufgeprägt, wenn der Kolben 4 sich entsprechend den Doppelpfeilen in Figur 1 hin- und herbewegt.

Erfindungsgemäß wird der in Figur 1 gezeigte Kompressor dazu verwendet einerseits einen Druckmittelstrom für die Erzeugung eines Aerosols zu erzeugen und andererseits Druckschwankungen in einem Gasvolumen bereitzustellen, die einer Aerosolhauptströmung überlagert werden können, die mit Hilfe des kontinuierlichen Druckmittelstroms erzeugt wird.

Um ein Inhalationstherapiegerät entsprechend anzuschließen, besitzt die Gasauslasseinrichtung 3 vorzugsweise einen Anschlussstutzen 3a, so dass beispielsweise eine Schlauchleitung zu einer Verneblerdüse in einem Inhalationstherapiegerät anschließbar ist. Ferner ist an der Gasdurchtrittsöffnung 6 der zweiten Kompressionskammer 5 vorteilhaft ein Anschlussstutzen 6a für eine Schlauchleitung vorgesehen, mit der die Druckschwankungen an das Inhalationstherapiegerät herangeführt werden, um dort der Aerosolströmung überlagert zu werden.

Durch die erfindungsgemäße Integration der Bewegungseinrichtung in die eine Kompressionskammer, in Figur 1 die zweite Kompressionskammer 5, wird ein kompakter Kompressor realisiert, der die beiden gewünschten Druckmittelquellen in einer Baueinheit bereitstellt. Ein einziger Elektromotor (nicht dargestellt) ist erforderlich, um die beiden Kompressoreinrichtungen anzutreiben, wobei vorteilhaft ausgenutzt wird, dass der Elektromotor den Kolben 4 des erfindungsgemäßen Kompressors mit einer Frequenz antreiben kann, durch die Druckschwankungen in jedem therapeutisch gewünschten Frequenzbereich erzeugt werden. Gleichzeitig wird eine ausreichende Druckmittelströmung mit Hilfe des ersten Kompressionsraums und der Gaseinlass- und - auslasseinrichtung 2 bzw. 3 erzeugt.

Durch eine geeignete Abstimmung der Materialien des Pleuels 7a und der Exzenter-Scheibe 7b kann erreicht werden, dass ein reibungs- und damit verlustarmer Betrieb möglich ist, ohne dass kontaminierende Abrieb- oder Schmierstoffe in dem Kompressionsraum vorhanden sind, der die Bewegungseinrichtung erfindungsgemäß aufnimmt. Dies ist erforderlich, da die in dem Kompressionsraum hervorgerufenen Druckschwankungen einer Aerosolströmung aufgeprägt werden, die einem Patienten zu therapeutischen Zwecken zugeführt wird. Eine Kontaminierung muss deshalb in jedem Fall unterbleiben. Geeignete Materialien für das Pleuel sind verschiedene Kunststoffe. Ein geeignetes Material für die Exzenter-Scheibe ist Zink.

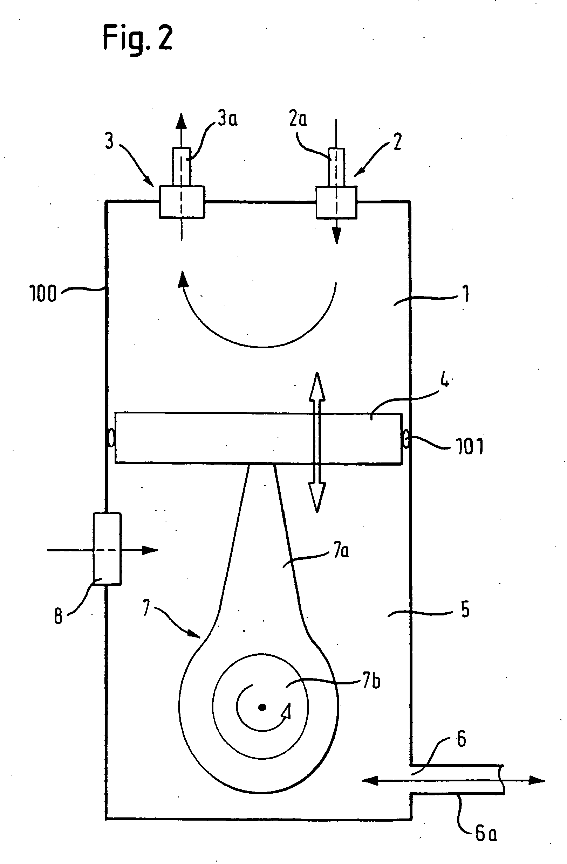

In Figur 2 ist ein weiteres Ausführungsbeispiel eines erfindungsgemäßen Kompressors für Inhalationstherapiegeräte gezeigt, das im Wesentlichen dem ersten Ausführungsbeispiel entspricht, so dass auf die obige Beschreibung des ersten Ausführungsbeispiels Bezug genommen wird. In Figur 2 sind deshalb auch die selben Bezugszeichen verwendet worden. In Abwandlung des ersten Ausführungsbeispiels ist bei dem zweiten Ausführungsbeispiel eine Gaseintrittseinrichtung 8 vorgesehen, die es gestattet, dass kontrolliert das zweite Gas, beispielsweise Umgebungsluft, in die zweite Kompressionskammer 5 eindringt, wenn es bei dem mit Druckschwankungen versehenen Gasvolumen zu Abflüssen bzw.

Verlusten kommt. Die Kontrolle des Eintritts des zweiten Gases durch die Gaseintrittseinrichtung 8 erfolgt beispielsweise durch ein vorgespanntes Eintrittsventil, das weiter unten noch beschrieben wird. Kommt es bei dem zweiten Ausführungsbeispiel im Hinblick auf das mit Druckschwankungen belegte Gasvolumen zu beabsichtigten Abflüssen des Gases oder zu Gasverlusten, wird bei einem durch die Vorspannung des Eintrittsventils ab einem vorgegebenen Druck sichergestellt, dass das zweite Gas, beispielsweise Umgebungsluft, in die zweite Kompressionskammer 5 nachströmen und den Abfluss bzw. Verlust ausgleichen kann.

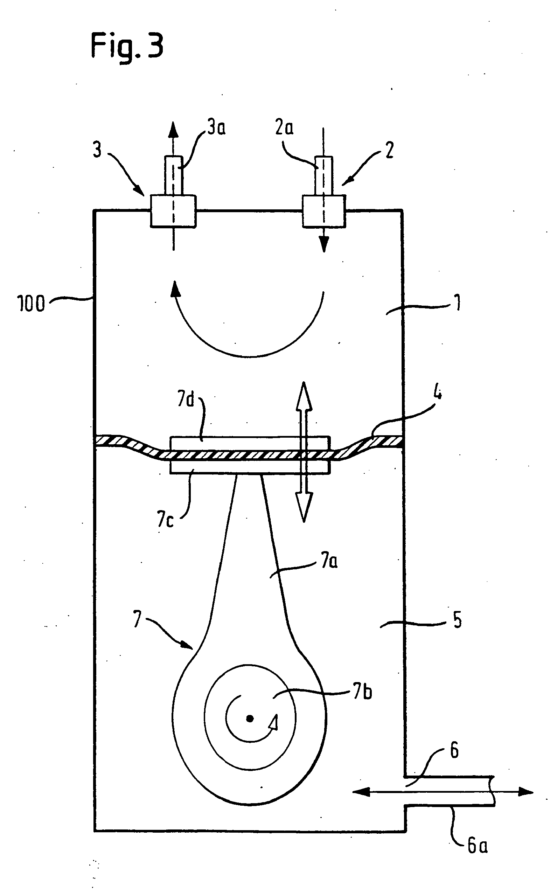

In Figur 3 ist ein drittes Ausführungsbeispiel eines erfindungsgemäßen Kompressors gezeigt, das im Wesentlichen dem Aufbau des ersten Ausführungsbeispiels entspricht, so dass auf die obige Beschreibung im Hinblick auf Figur 1 Bezug genommen wird. In Figur 3 wurden auch dieselben Bezugszeichen verwendet. In Abwandlung zum ersten Ausführungsbeispiel ist bei dem Kompressor gemäß dem dritten Ausführungsbeispiel als Kompressionseinrichtung 4 eine Membran vorgesehen, die an dem Gehäuse befestigt ist und das Gehäuse 100 in den ersten und zweiten Kompressionsraum 1 bzw. 5 unterteilt. Die Membran ist eingeklemmt zwischen zwei Scheiben 7c und 7d, die als Teil der Bewegungseinrichtung 7 betrachtet werden können. Werden die beiden Scheiben 7c und 7d durch das Pleuel 7a entsprechend den Doppelpfeilen in Figur 3 hin- und herbewegt, wird auch die Membran aus ihrer Ruhelage ausgelenkt, so dass es zu den Kompressions- und Expansionsbewegungen in Bezug auf die erste Kompressionskammer 1 bzw. die zweite Kompressionskammer 5 kommt. Im übrigen entspricht die Funktionsweise des dritten Ausführungsbeispiels vollständig dem ersten Ausführungsbeispiel, so dass erneut im Hinblick auf die Funktionsweise Bezug genommen wird auf die Schilderungen im Zusammenhang mit dem ersten Ausführungsbeispiel.

Ferner ist offensichtlich, dass auch die Gaseintrittseinrichtung 8, die in Zusammenhang mit dem zweiten Ausführungsbeispiel geschildert wurde, bei einem Kompressor gemäß dem dritten Ausführungsbeispiel vorgesehen werden kann.

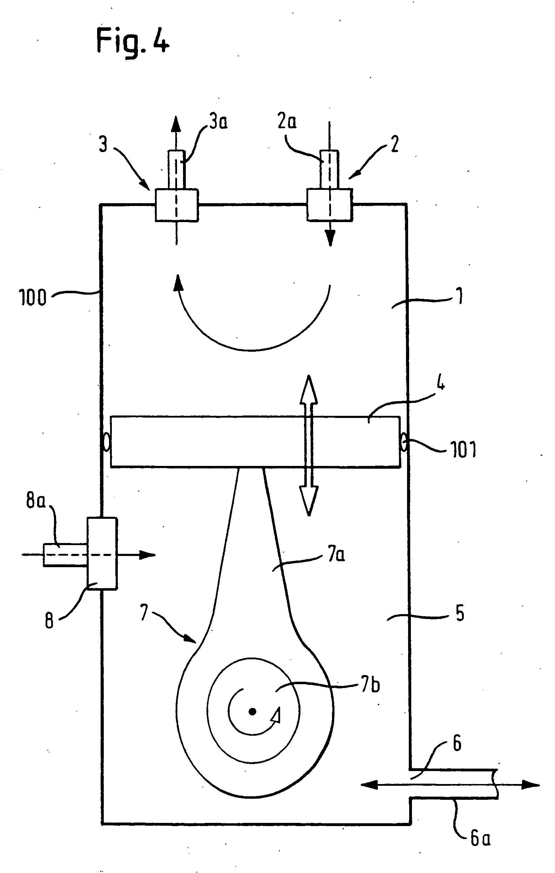

In Figur 4 ist ein viertes Ausführungsbeispiel eines erfindungsgemäßen Kompressors gezeigt, das im wesentlichen dem Aufbau des zweiten Ausführungsbeispiels entspricht, so dass auf die obige Beschreibung im Hinblick auf Figur 2 Bezug genommen wird. In Figur 4 wurden auch die selben Bezugszeichen verwendet. In Abwandlung zum zweiten Ausführungsbeispiel umfasst die Gaseintrittseinrichtung 8 einen Anschluss 8a für eine Schlauchleitung, so dass als zweites Gas ein therapeutisch oder diagnostisch wirksames Gas der zweiten Kompressionskammer 5 zugeführt werden kann. Auch in diesem Fall tritt das therapeutisch oder diagnostisch wirksame Gas durch die Gaseintrittseinrichtung 8 in kontrollierter Form zu, beispielsweise erst ab einem bestimmten Druck, was durch ein bereits erwähntes vorgespanntes Gaseintrittsventil bewerkstelligt werden kann.

An dieser Stelle sei angemerkt, dass bei allen Ausführungsbeispielen an die Gaseinlasseinrichtung 2 ebenfalls eine Schlauchleitung angeschlossen werden kann, die ein therapeutisch oder diagnostisch wirksames Gas oder Gas-Luft-Gemisch zuführt, das mit Hilfe des erfindungsgemäßen Kompressors als kontinuierliche Druckmittelströmung am Anschlussstutzen der Gasauslasseinrichtung 3 bereitgestellt wird. Dazu umfasst die Gaseinlasseinrichtung 2 beispielsweise einen Anschlussstutzen 2a.

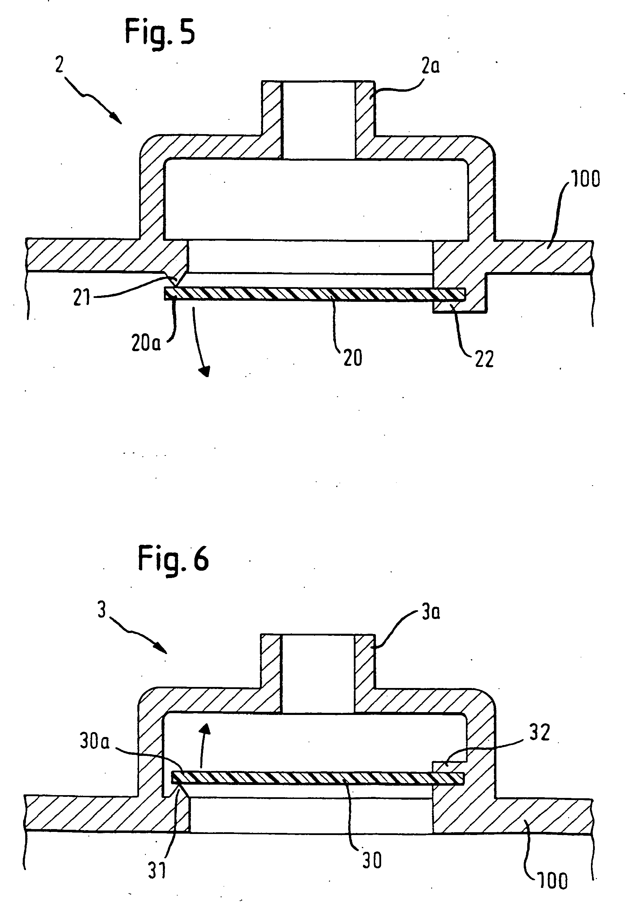

In Figur 5 ist beispielhaft ein Einlassventil dargestellt, das sich für die Anordnung in der Gaseinlasseinrichtung 2 eines erfindungsgemäßen Inhalationstherapiegerätekompressors eignet. Das in Figur 5 beispielhaft gezeigte Einlassventil umfasst ein Ventilelement 20, das in Bezug auf einem Ventilsitz 21 mit einer Befestigungseinrichtung 22 derart befestigt ist, dass es sich nur an seinem freien Ende 20a vom Ventilsitz 21 ablösen kann, wenn das erste Gas, beispielsweise Umgebungsluft oder das oben angesprochene Gas-Luft-Gemisch, der Gaseinlasseinrichtung zuströmt. Dies geschieht, wenn die Kompressionseinrichtung 4 des erfindungsgemäßen Kompressors in der ersten Kompressionskammer 1 einen Unterdruck erzeugt.

Als Gegenstück zu dem in Figur 5 gezeigten Einlassventil ist in Figur 6 ein Auslassventil dargestellt, das in der Gasauslasseinrichtung 3 des erfindungsgemäßen Kompressors vorgesehen ist und das ein Ventilelement 30 umfasst, das an einem Ventilsitz 31 anliegt und von einer Befestigung 32 gehalten wird. Erzeugt die Kompressionseinrichtung 4 in der Kompressionskammer 1 einen Überdruck, wird das freie Ende 30a des Ventilelements 30 des Auslassventils vom Ventilsitz 31 abgehoben, so dass das komprimierte Gas oder Gas-Luft-Gemisch durch die Gasauslasseinrichtung 3 austreten kann.

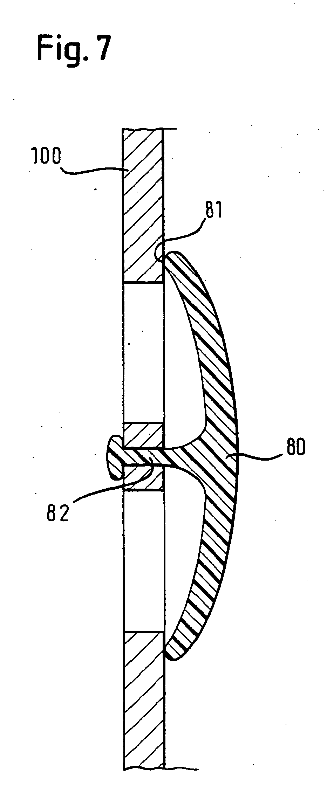

In Figur 7 ist beispielhaft ein vorgespanntes Eintrittsventil gezeigt, das in der Gaseintrittseinrichtung 8 vorgesehen ist, die im Bezug auf die zweite Kompressionskammer 5 in einem erfindungsgemäßen Kompressor vorgesehen sein kann. Das Ventilelement 80 hat eine gewölbte kreisförmige Grundform und ist mit Hilfe eines Befestigungszapfens 82 bezogen auf einen Ventilsitz 81 fixiert. Wenn der Unterdruck in der zweiten Kompressionskammer 5 aufgrund der Bewegung der Kompressionseinrichtung 4 und aufgrund von Abflüssen bzw. Verlusten des Gasvolumens einen Wert unterschreitet, der der Vorspannung entspricht, strömt das zweite Gas, beispielsweise Luft oder das oben erwähnte Gas-Luft-Gemisch in die zweite Kompressionskammer 5 nach.

Wie bereits eingangs erwähnt kann die Bewegungseinrichtung 7, die in den geschilderten vier Ausführungsbeispielen im zweiten Kompressionsraum 5 angeordnet ist, im ersten Kompressionsraum 1 angeordnet werden, sofern der erste Kompressionsraum ausreichend dicht gestaltet wird. Dabei ist zu beachten, dass die erzielten Drücke und Fördervolumina im Vergleich mit dem zweiten Kompressionsraum sehr viel höher sind, so dass an die Dichtigkeit des ersten Kompressionsraums andere, höhere Ansprüche gestellt werden, als an die Dichtigkeit des zweiten Kompressionsraums. Sofern die Dichtigkeit aber durch geeignete, dem Fachmann bekannte technische Maßnahmen gewährleistet wird, steht der Anordnung der Bewegungseinrichtung im ersten Kompressionsraum nichts entgegen.

Ein erfindungsgemäßer Kompressor kann nicht nur in Verbindung mit Inhalationstherapiegeräten verwendet werden, die eine Verneblerdüse aufweisen, sondern auch mit anderen Verneblern, beispielsweise Membran-Verneblern, wie er beispielsweise in EP 1 304 130 A beschrieben wird. In diesem Fall wird die kontinuierliche Druckmittelströmung nicht zur Erzeugung des Aerosols, wohl aber zur Erzeugung einer Aerosolgrundströmung verwendet, in dem das von dem Membran- oder Ultraschall-Vernebler erzeugte Aerosol in die Druckmittelströmung gemischt wird. Die mit Hilfe des erfindungsgemäßen Kompressors erzeugten Druckschwankungen werden dann der so erzeugten Aerosolgrundströmung aufgeprägt.

## Patentansprüche

1. Inhalationstherapiegerätekompressor für Inhalationstherapiegeräte zur Erzeugung eines kontinuierlichen Druckmittelstroms und zur Erzeugung von Druckschwankungen mit
- einem ersten Kompressionsraum (1), der für die Erzeugung des kontinuierlichen Druckmittelstroms ausgelegt ist;
- einer Gaseinlasseinrichtung (2) für das Einströmen eines ersten Gases in den ersten Kompressionsraum (1) ;
- einer Gasauslasseinrichtung (3) für das Ausströmen des ersten Gases aus dem ersten Kompressionsraum (1);
- einer Kompressionseinrichtung (4), die den ersten Kompressionsraum (1) derart abschließt, dass bei Bewegung der Kompressionseinrichtung (4) das erste Gas über die Gaseinlasseinrichtung (2) in den ersten Kompressionsraum (1) hinein und über die Gasauslasseinrichtung (3) aus dem ersten Kompressionsraum (1) heraus gefördert wird;
- einem zweiten Kompressionsraum (5), der für die Erzeugung der Druckschwankungen ausgelegt ist;
- einer Gasdurchtrittsöffnung (6) für das Ein- und Austreten eines zweiten Gases in den bzw. aus dem zweiten Kompressionsraum (5);
- einer Bewegungseinrichtung (7), die äußerhalb des ersten Kompressionsraumes (1) und innerhalb des zweiten Kompressionsraumes (5) angeordnet ist und die mit der Kompressionsreinrichtung (4) derart verbunden ist, dass die Kompressionseinrichtung (4) durch die Bewegungseinrichtung (7) bewegbar ist.

2. Inhalationstherapiegerätekompressor nach Anspruch 1, **dadurch gekennzeichnet, dass** die Gaseinlasseinrichtung (2) ein Einlassventil (20, 20a, 21, 22) umfasst.

3. Inhalationstherapiegerätekompressor nach Anspruch 2, **dadurch gekennzeichnet, dass** an der Gaseinlasseinrichtung (2) ein Anschluss (2a) für die Zuführung des ersten Gases zu dem Kompressor vorgesehen ist.

4. Inhalationstherapiegerätekompressor nach Anspruch 3, **dadurch gekennzeichnet, dass** der Anschluss ein Anschlussstutzen (2a) für eine Schlauchleitung ist.

5. Inhalationstherapiegerätekompressor nach einem der Ansprüche 1 bis 4, **dadurch gekennzeichnet, dass** die Gasauslasseinrichtung (3) ein Auslassventil (30, 30a, 31, 32) umfasst.

6. Inhalationstherapiegerätekompressor nach Anspruch 5, **dadurch gekennzeichnet, dass** an der Gasauslasseinrichtung (3) ein Anschluss (3a) für die Abführung des ersten Gases von dem Kompressor vorgesehen ist.

7. Inhalationstherapiegerätekompressor nach Anspruch 6, **dadurch gekennzeichnet, dass** der Anschluss ein Anschlussstutzen (3a) für eine Schlauchleitung ist.

8. Inhalationstherapiegerätekompressor nach einem der Ansprüche 1 bis 7, **dadurch gekennzeichnet, dass** eine Gaseintrittseinrichtung (8) vorgesehen ist, die den kontrollierten Eintritt des zweiten Gases in den zweiten Kompressionsraum (5) gestattet.

9. Inhalationstherapiegerätekompressor nach Anspruch 8, **dadurch gekennzeichnet, dass** die Gaseintrittseinrichtung (8) ein Eintrittsventil (80, 81, 82) umfasst.

10. Inhalationstherapiegerätekompressor nach Anspruch 8 oder 9, **dadurch gekennzeichnet, dass** an der Gaseintrittseinrichtung (8) ein Anschluss (8a) für die Zuführung des zweiten Gases zu dem Kompressor vorgesehen ist.

11. Inhalationstherapiegerätekompressor nach Anspruch 10, **dadurch gekennzeichnet, dass** der Anschluss ein Anschlussstutzen (8a) für eine Schlauchleitung ist.

12. Inhalationstherapiegerätekompressor nach einem der Ansprüche 1 bis 11, **dadurch gekennzeichnet, dass** die Kompressionseinrichtung (4) ein Kolben ist.

13. Inhalationstherapiegerätekompressor nach einem der Ansprüche 1 bis 11, **dadurch gekennzeichnet, dass** die Kompressionseinrichtung (4) eine Membran ist.

14. Inhalationstherapiegerätekompressor nach einem der Ansprüche 1 bis 13, **dadurch gekennzeichnet, dass** die Bewegungseinrichtung ein Pleuel (7a) und eine Exzenter-Scheibe (7b) umfasst.

15. Inhalationstherapiegerätekompressor nach einem der Ansprüche 1 bis 14, **dadurch gekennzeichnet, dass** das erste Gas und/oder das zweite Gas Luft oder ein therapeutisch wiksames Gas oder Gas-Luft-Gemisch ist.

## Claims

1. Inhalation therapy device compressor for inhalation therapy devices for producing a continuous pressure medium flow and for producing pressure variations, having
- a first compressor space (1) which is designed to produce the continuous pressure medium flow;
- a gas inlet means (2) for the inflow of a first gas into the first compression space (1);
- a gas outlet means (3) for the outflow of the first gas from the first compression space (1);
- a compression means (4) that closes off the first compression space (1) in such a way that, when the compression means (4) moves, the first gas is conveyed into the first compression space (1) via the gas inlet means (2) and conveyed out of the first compression space (1) via the gas outlet means (3);
- a second compression space (5) which is designed for the production of the pressure variations;
- a gas passage opening (6) for the entry and escape of a second gas into or out of the second compression space (5); and
- a movement means (7) which is arranged outside the first compression chamber (1) and within the second compression space (5) and which is associated with the compression means (4) in such a way that the compression means (4) can be moved by the movement means (7).

2. Inhalation therapy device compressor according to claim 1, charactarised that the gas inlet device (2) comprises an inlet valve (20, 20a, 21, 22).

3. Inhalation therapy device compressor according to claim 2, charactarised that the gas inlet device (2) is provided with a connection (2a) for supplying the first gas to the compressor.

4. Inhalation therapy device compressor according to claim 3, charactarised in that the connection is a connecting sleeve (2a) for a hose line.

5. Inhalation therapy device compressor according to one of the claims 1 to 4, charactarised in that the gas outlet means (3) comprises an outlet valve (30, 30a, 31, 32).

6. Inhalation therapy device compressor according to claim 5, charactarised in that the gas outlet means (3) is provided with a connection (3a) for the removal of the first gas from the compressor.

7. Inhalation therapy device compressor according to claim 6, charactarised in that the connection is a connecting sleeve (3a) for a hose line.

8. Inhalation therapy device compressor according to one of the claims 1 to 7, charactarised in that a gas inlet means (8) is provided that permits the controlled entry of the second gas into the second compression space (5).

9. Inhalation therapy device compressor according to claim 8, charactarised in that a gas inlet means (8) comprises an inlet valve (80, 81, 82).

10. Inhalation therapy device compressor according to claim 8 or 9, charactarised in that the gas inlet means (8) is provided with a connection (8a) for the supply of a second gas to the compressor.

11. Inhalation therapy device compressor according to claim 10, charactarised in that the connection is a connecting sleeve (8a) for a hose line.

12. Inhalation therapy device compressor according to one of the claims 1 to 11, charactarised in that the compression means (4) is a piston.

13. Inhalation therapy device compressor according to one of the claims 1 to 11, charactarised in that the compression means (4) is a diaphragm.

14. Inhalation therapy device compressor according to one of the claims 1 to 13, charactarised in that the movement means comprises a connecting rod (a) and an eccentric plate (7b).

15. Inhalation therapy device compressor according to one of the claims 1 to 14, charactarised in that the first gas and/or the second gas is air or a therapeutically active gas or a gas/air mixture.

## Revendications

1. Compresseur d'appareils de thérapie par inhalation, pour des appareils de thérapie par inhalation pour produire un flux de fluide sous pression continu et pour produire des fluctuations de pression, avec :
- une première enceinte de compression (1), conçue pour produire le flux de fluide sous pression continu ;
- un dispositif d'admission de gaz (2), pour l'introduction d'un flux d'un premier gaz dans la première enceinte de compression (1) ;
- un dispositif d'échappement de gaz (3), pour l'échappement du premier gaz hors de l'enceinte de compression (1) ;
- un dispositif de compression (4), fermant la première enceinte de compression (1), de manière que, en cas de déplacement du dispositif de compression (4), le premier gaz soit transféré, par le dispositif d'admission de gaz (2), dans la première enceinte de compression (1) et, par le dispositif d'échappement de gaz (3), soit refoulé hors de la première enceinte de compression (1) ;
- une deuxième enceinte de compression (5), conçue pour produire les fluctuations de pression ;
- une ouverture de passage de gaz (6), pour l'entrée et la sortie d'un deuxième gaz, dans ou hors de la deuxième enceinte de compression (5) ;
- un dispositif de déplacement (7), disposé à l'extérieur de la première enceinte de compression (1) et à l'intérieur de la deuxième enceinte de compression (5) et relié au dispositif de compression (4), de manière que le dispositif de compression (4) soit déplaçable au moyen du dispositif de déplacement (7).

2. Compresseur d'appareils de thérapie par inhalation selon la revendication 1, **caractérisé en ce que** le dispositif d'admission de gaz (2) comprend une soupape d'admission (20, 20a, 21, 22)

3. Compresseur d'appareils de thérapie par inhalation selon la revendication 2, **caractérisé en ce qu'**un raccordement (2a) pour l'amenée du premier gaz au compresseur est prévu sur le dispositif d'admission de gaz (2).

4. Compresseur d'appareils de thérapie par inhalation selon la revendication 3, **caractérisé en ce que** le raccordement est une tubulure de raccordement (2a) pour une conduite en tuyau.

5. Compresseur d'appareils de thérapie par inhalation selon l'une des revendications 1 à 4, **caractérisé en ce que** le dispositif d'échappement de gaz (3) comprend une soupape d'échappement (30, 30a, 31, 32).

6. Compresseur d'appareils de thérapie par inhalation selon la revendication 5, **caractérisé en ce qu'**un raccordement (3a) pour l'échappement du premier gaz hors du compresseur est prévu sur le dispositif d'échappement de gaz (3).

7. Compresseur d'appareils de thérapie par inhalation selon la revendication 6, **caractérisé en ce que** le raccordement est une tubulure de raccordement (3a) pour une conduite en tuyau.

8. Compresseur d'appareils de thérapie par inhalation selon l'une des revendications 1 à 7, **caractérisé en ce qu'**est prévu un dispositif d'entrée de gaz (8), permettant l'entrée contrôlée du deuxième gaz dans la deuxième enceinte de compression (5).

9. Compresseur d'appareils de thérapie par inhalation selon la revendication 8, **caractérisé en ce que** le dispositif d'entrée de gaz (8) comprend une soupape d'entrée (80, 81, 82).

10. Compresseur d'appareils de thérapie par inhalation selon la revendication 8 ou 9, **caractérisé en ce qu'**un raccordement (8a) pour l'amenée du deuxième gaz au compresseur est prévu sur le dispositif d'entrée de gaz (8).

11. Compresseur d'appareils de thérapie par inhalation selon la revendication 10, **caractérisé en ce que** le raccordement est une tubulure de raccordement (8a) pour une conduite en tuyau.

12. Compresseur d'appareils de thérapie par inhalation selon l'une des revendications 1 à 11, **caractérisé en ce que** le dispositif de compression (4) est un piston.

13. Compresseur d'appareils de thérapie par inhalation selon l'une des revendications 1 à 11, **caractérisé en ce que** le dispositif de compression (4) est une membrane.

14. Compresseur d'appareils de thérapie par inhalation selon l'une des revendications 1 à 13, **caractérisé en ce que** le dispositif de déplacement est une bielle (7a) et comprend un disque à excentrique (7b).

15. Compresseur d'appareils de thérapie par inhalation selon l'une des revendications 1 à 14, **caractérisé en ce que** le premier gaz et/ou le deuxième gaz est de l'air, ou un gaz ou mélange gaz-air, thérapeutiquement actif.
